# EUROPEAN PATENT APPLICATION

(11) **EP 4 667 481 A1**
(43) Date of publication of application: **24.12.2025**
(21) Application number: 24756938.7
(22) Date of filing: 15.02.2024
(51) Int. Cl.: C07K 1/14, C07K 1/16, C07K 14/46, A61K 31/737, A61L 27/20, A61L 27/22, A61L 27/36

(54) **AGENT FOR REDUCING ENDOTOXIN IN PROTEOGLYCANS DERIVED FROM ANIMAL TISSUES, AND METHOD FOR PRODUCING PROTEOGLYCAN**

(30) Priority: 16.02.2023 JP 2023022139; 03.08.2023 JP 2023127199; 30.11.2023 JP 2023203052
(71) Applicant: National University Corporation Tottori University, Tottori-shi, Tottori 680-8550 (JP); Ichimaru Pharcos Co., Ltd., Motosu-shi, Gifu 501-0475 (JP)
(72) Inventor: TAMURA Junichi, Tottori-shi, Tottori 680-8550 (JP); NAKAJIMA, Motowo, Motosu-shi, Gifu 501-0475 (JP); MASUTANI Teruaki, Motosu-shi, Gifu 501-0475 (JP); TAKAHASHI Tatsuji, Motosu-shi, Gifu 501-0475 (JP)
(74) Representative: Hasegawa, Kan
(86) International application number: PCT/JP2024/005141
(87) International publication number: WO 2024/172098

(57) **Abstract**

The present disclosure provides an agent capable of reducing endotoxin and used for reducing endotoxin in animal tissue-derived proteoglycan, and a method for producing proteoglycan and capable of reducing endotoxin. The agent of the present disclosure for reducing endotoxin in the animal tissue-derived proteoglycan comprises a nonionic surfactant.

## Description

### TECHNICAL FIELD

The present disclosure relates to an agent for reducing endotoxin in proteoglycan derived from a tissue of an animal and a method for producing proteoglycan.

### BACKGROUND ART

In the regenerative medicine market, sheet-shaped tissues for regenerative medicine are used in most cases. However, it is difficult to regenerate a tissue having a three dimensional structure with the sheet-shaped tissue. Thus, attempts have been made to induce the three dimensional tissue. When constructing the three dimensional tissue, a problem is the difficulty in reproducing the same structures of intercellular crosstalk and extracellular matrix as those in vivo. For this reason, development of a material capable of reproducing the structure of the extracellular matrix has been desired (Non-Patent Document 1).

For use in animals comprising humans, the tissues for regenerative medicine are required to have endotoxin (endotoxin) reduced and to be approved by standards for materials of biological origin (Patent Document 1).

### CITATION LIST

### NON-PATENT DOCUMENT

Non-Patent Document 1: Akagi Takami, Akashi Mitsuru "Construction of Three-Dimensional Tissue Models by Cell Manipulation for Drug Development and Regenerative Medicine" Journal of Pharmaceutical Science and Technology, Japan 76.5 (2016): 294-300.

### PATENT DOCUMENT

Patent Document 1: International Patent Publication No. WO 2017/018524

### SUMMARY OF THE INVENTION

### TECHNICAL PROBLEM

The present inventors have conceived of that proteoglycan can be used as the material because the extracellular matrix is rich in proteoglycan. For use of proteoglycan for medical purposes, the present inventors removed endotoxin in proteoglycan by a method for removing endotoxin using a basic organic solvent. This method can remove endotoxin, but has drawbacks, for example, a predetermined molecular weight of proteoglycan cannot be maintained.

Thus, in a first aspect, the present disclosure provides, for example, a method for producing proteoglycan capable of reducing endotoxin and maintaining a predetermined molecular weight of proteoglycan.

In a second aspect, the present disclosure provides, for example, an agent capable of reducing endotoxin and used for reducing endotoxin in proteoglycan derived from a tissue of an animal, and a method for producing proteoglycan and capable of reducing endotoxin.

### SOLUTION TO THE PROBLEM

In the first aspect of the present disclosure, the method for producing proteoglycan comprises a reduction step of reducing endotoxin in proteoglycan derived from a tissue of an animal by using a nonionic surfactant.

A composition of the present disclosure comprises proteoglycan derived from a tissue of an animal. The composition comprises the proteoglycan and endotoxin, and a content ratio of the endotoxin to the composition is 4 EU/mg or less.

In the second aspect of the present disclosure, the agent for reducing endotoxin in proteoglycan derived from a tissue of an animal comprises a nonionic surfactant.

The method for producing proteoglycan of the present disclosure comprises a first reduction step of reducing endotoxin in proteoglycan derived from a tissue of an animal by using a nonionic surfactant, and a second reduction step of treating the proteoglycan with a chromatography carrier, thereby reducing endotoxin in the proteoglycan.

The present disclosure can provide, for example, a method for producing proteoglycan and capable of reducing endotoxin and maintaining a predetermined molecular weight of proteoglycan. The present disclosure can provide, for example, an agent capable of reducing endotoxin and used for reducing endotoxin in proteoglycan derived from a tissue of an animal, and a method for producing proteoglycan and capable of reducing endotoxin.

### BRIEF DESCRIPTION OF THE DRAWINGS

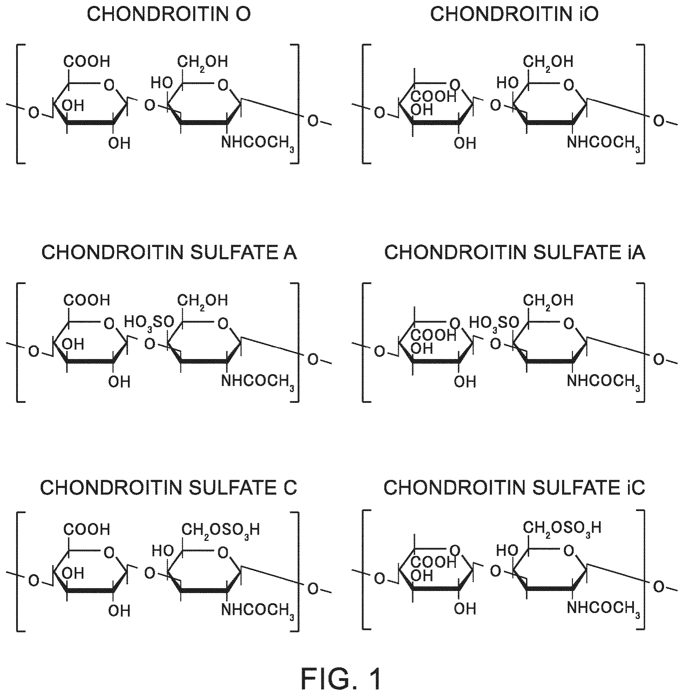

FIG. 1 illustrates main disaccharide structures of chondroitin sulfates.

### DESCRIPTION OF EMBODIMENTS

The present disclosure will be described in detail below by way of examples. Hereinafter, unless otherwise specified, each example can be incorporated by reference into the description of other examples.

### <Definitions>

As used herein, "proteoglycan" refers to a molecule (glycoprotein) in which a protein (a core protein) and glycosaminoglycans (GAGs, also referred to as "polysaccharides" or "sugar chains") are covalently bonded. The proteoglycan exists in, for example, an extracellular matrix of skin, organs, and cartilage. The glycosaminoglycans are generally known as sugar chains having a long-chain structure with no branches. Examples of the proteoglycan comprise: aggrecan family (also referred to as lectican family or hyalectan family) such as aggrecan, versican, neurocan, and brevican; small leucine-rich proteoglycans (SLRPs) family such as biglycan, decorin, fibromodulin, lumican, PG-Lb (epiphycan), keratocan, and mimecan; proteoglycans of a basement membrane such as perlecan, agrin, and bamacan; and other proteoglycans such as testican, biglycan, serglycin, syndecan, dystroglycan, claustrin, glypican, and keratocan. The proteoglycans can be classified into chondroitin sulfate proteoglycans, dermatan sulfate proteoglycans, heparan sulfate proteoglycans, and keratan sulfate proteoglycans depending on, for example, the type of GAGs bonded to the protein.

Examples of the GAGs comprise chondroitin, chondroitin sulfate (CS), dermatan sulfate (DS, chondroitin sulfate B), heparan sulfate, heparin, and keratan sulfate. Examples of the chondroitin comprise an O-sugar chain having a main disaccharide structure of glucuronic acid and acetylgalactosamine, and an iO-sugar chain having a main disaccharide structure of iduronic acid and acetylgalactosamine (they may also be referred to as "chondroitin sulfate O" and "chondroitin sulfate iO"). Chondroitin sulfate (CS) is composed of a sugar chain of two repeating sugars, glucuronic acid and acetylgalactosamine, and a sulfate group added to the sugar chain. Examples of the chondroitin sulfate (CS) comprise chondroitin sulfate A (type A) having a main disaccharide structure of glucuronic acid and acetylgalactosamine-4-sulfate, and chondroitin sulfate C (type C) having a main disaccharide structure of glucuronic acid and acetylgalactosamine-6-sulfate. Dermatan sulfate (DS) is composed of a sugar chain of two repeating sugars, iduronic acid and acetylgalactosamine, and a sulfate group added to the sugar chain. Examples of dermatan sulfate comprise chondroitin sulfate iA (type iA) having a main disaccharide structure of iduronic acid and acetylgalactosamine-4-sulfate, and chondroitin sulfate iC (type iC) having a main disaccharide structure of iduronic acid and acetylgalactosamine-6-sulfate. The chondroitin sulfates each have, for example, a main disaccharide structure shown in FIG. 1. In FIG. 1, the sulfate group (sulfo group) is bonded to a hydrogen atom, but the present disclosure is not limited to this example. The sulfate group attached to the GAG may be ionized by elimination of a hydrogen atom, or may form a salt, for example.

As used herein, "endotoxin (endotoxin)" refers to a toxic component derived from lipopolysaccharide (LPS), which is a cell wall component of Gram-negative bacteria. Endotoxin is released, for example, when the Gram-negative bacteria dies and the cell wall of the Gram-negative bacteria breaks. Endotoxin is known to cause biological reactions such as fever, septic shock, and multiple organ failure when entering the blood of humans or other organisms. Endotoxin is composed of an O-antigen polysaccharide, a core polysaccharide, and lipid A. Endotoxin has a hydrophilic moiety (sugar parts of the O-antigen polysaccharide and the core polysaccharide) and a hydrophobic moiety (lipid A), and can cause association in an aqueous solution to form a micelle.

As used herein, a "chromatography carrier" means any type of a stationary phase that separates particular molecules from other molecules present in a mixture. Examples of the stationary phase used as the chromatography carrier comprise a resin, a porous material such as a monolith (e.g., a silica monolith), and a membrane. When the stationary phase is a resin, the chromatography carrier can be referred to as a chromatography resin.

In the present specification, a "micelle" means a spherical structure made up of amphiphilic molecules that each have a hydrophobic moiety and a hydrophilic moiety and are aggregated in an aqueous solvent such as water, with the hydrophilic moiety in contact with the aqueous solvent and the hydrophobic moiety facing inward. The concentration at which the micelles are formed from a molecular dispersion is referred to as a critical micelle concentration (CMC) of a material.

In the present specification, a "surfactant" means a compound having a hydrophilic group and a hydrophobic group (lipophilic group). The surfactant forms the micelles at or above the critical micelle concentration. Examples of the surfactant comprise an anionic surfactant, a cationic surfactant, an amphoteric surfactant, and a nonionic surfactant. The anionic surfactant means a surfactant which dissociates into anions in an aqueous solution. The cationic surfactant means a surfactant which dissociates into cations in an aqueous solution. The amphoteric surfactant means a surfactant which dissociates into cations or anions in an aqueous solution depending on the pH of the aqueous solution. The nonionic surfactant means a surfactant which does not dissociate into ions in an aqueous solution.

In the present specification, a "cloud point (or clouding point)" means a temperature at which a solution comprising a nonionic surfactant starts to become cloudy when heated (phase transition temperature). When heated, the nonionic surfactant rapidly decreases in water solubility, and the nonionic surfactant cannot form the micelles, turning cloudy. The water solubility rapidly decreases when polyether chains of the nonionic surfactant fail to form hydrogen bonds with water. The cloud point can be measured, for example, by visual observation, measurement according to a measurement standard for determination of the cloud point of a nonionic surfactant (ISO 1065:1991), or a method described in Japanese Unexamined Patent Publication No. 2006-257395. The method of Japanese Unexamined Patent Publication No. 2006-257395 is as follows.
- The method is described at page 95 of "New Introduction to Surfactants" by Takehiko Fujimoto (Sanyo Chemical Industries, Ltd.). In this method, a 1 mass% aqueous nonionic compound solution is placed in a test tube with a thermometer and a stirrer, and slowly heated while gently stirring with the stirrer. The solution which is transparent becomes cloudy when the temperature reaches or exceeds a certain level, and the temperature at that time is measured and determined as the cloud point.

For example, JIS K 3211 also describes the definition of the cloud point of surfactants.

As used herein, an "ion exchange carrier" (ion exchanger) means a solid phase that is positively or negatively charged and has free anions or free cations for exchange with anions or cations in a liquid passing over or inside the solid phase. When the ion exchange carrier is positively charged and allows exchange with the anions, the ion exchange carrier may also be referred to as an anion exchange carrier or an anion exchanger. The anion exchange carrier has positively charged ligands, such as primary, secondary, tertiary or quaternary amino groups. When the ion exchange carrier is negatively charged and allows exchange with the cations, the ion exchange carrier can also be referred to as a cation exchange carrier or a cation exchanger.

In the present specification, a "hydrophobic interaction carrier" means a solid phase which has a hydrophobic group and adsorbs molecules comprised in a liquid passing over or inside the solid phase, depending on the hydrophobicity of the molecules. Examples of the hydrophobic group comprise the following functional groups: an alkyl group such as an octyl group and an octadecyl group; a functional group comprising an aryl group such as a phenyl group or a benzyl group; and a polyamine functional group such as polylysine (e.g., ε-polylysine).

As used herein, in a "flow through mode" (F/T mode), at least one substance to be removed from an object is bonded or adsorbed to a stationary phase such as a chromatography resin, while at least one target substance in the object flows through the stationary phase to be separated from the object. The F/T mode is also referred to as, for example, a negative mode.

As used herein, in a "bind/elute mode" (B/E mode), at least one target substance comprised in an object is bonded or adsorbed to a stationary phase such as a chromatography resin, and then the target substance is eluted to be separated from the object.

Hereinafter, the present disclosure will be described by way of examples, but the present disclosure is not limited to the following examples, and can be implemented with any modifications. The descriptions of the present disclosure and the embodiments can be mutually incorporated unless otherwise specified. In this specification, when the expression "to" is used between numerical or physical values, it means that the numerical or physical values before and after "to" are comprised. In the present specification, the expression "A and/or B" means "A alone," "B alone," and "both A and B."

### <Method for Producing Proteoglycan>

In a certain aspect, the present disclosure provides a method for producing proteoglycan. The method of the present disclosure comprises first reduction step of reducing endotoxin in proteoglycan derived from a tissue of an animal by using a nonionic surfactant. The method of the present disclosure can reduce endotoxin with a predetermined molecular weight of proteoglycan maintained.

As a result of intensive studies, the present inventors have conceived of that endotoxin in a composition comprising proteoglycan and endotoxin could be reduced by using the difference in hydrophobicity and hydrophilicity between proteoglycan which is a glycoprotein and endotoxin which is a glycolipid. As a result of further studies, the present inventors have found that endotoxin can be extracted from a mixture of proteoglycan and endotoxin by using a nonionic surfactant, and thus have achieved the present disclosure. In the present disclosure, it is presumed that endotoxin can be reduced by the mechanism described below using the nonionic surfactant. However, the present disclosure is not limited to the following mechanism. Proteoglycan is a hydrophilic component composed of sugars and proteins. On the other hand, endotoxin has a hydrophilic moiety and a hydrophobic moiety as described above. Thus, when the proteoglycan, the endotoxin, and the nonionic surfactant coexist in an aqueous solvent under a temperature condition that allows the nonionic surfactant to be present in a dispersed state in the aqueous solvent, the proteoglycan, the endotoxin, and the nonionic surfactant behave differently due to the difference in hydrophilicity and hydrophobicity. Specifically, the proteoglycan can be dispersed alone in the aqueous solvent. The endotoxin and the nonionic surfactant, which comprise the hydrophobic moiety, cannot be dispersed alone in the aqueous solvent, and gather together to form a complex. Thus, the endotoxin and the nonionic surfactant are present as the complex in the aqueous solvent. When the aqueous solvent in this state is heated to a temperature higher than the cloud point of the nonionic surfactant, the complex comprising the nonionic surfactant cannot remain dispersed in the aqueous solvent, and forms a phase of the nonionic surfactant (surfactant phase). The endotoxin, which is in the form of the complex with the nonionic surfactant, is concentrated (extracted) in the phase of the nonionic surfactant in the formation of the phase (phase separation). Thus, in the present disclosure, it is presumed that the amount of endotoxin in the composition comprising proteoglycan and endotoxin can be reduced by extracting the endotoxin from the proteoglycan-comprising composition into a different phase.

Examples of the nonionic surfactant comprise surfactants having polyether chains such as polyoxyethylene alkylphenyl ether (POEAE). The polyoxyethylene alkylphenyl ether is, for example, polyethylene glycol tert-octylphenyl ether (the following formula (1)). In the following formula (1), n represents the degree of polymerization of ethylene glycol. For example, n is preferably 1 to 10, more preferably 7 to 8. The nonionic surfactant is, for example, a nonionic surfactant that undergoes a phase transition in a temperature-dependent manner. For example, the surfactants may be used alone or in combination of two or more of them. When two or more surfactants are used, for example, the surfactants may be mixed in advance.

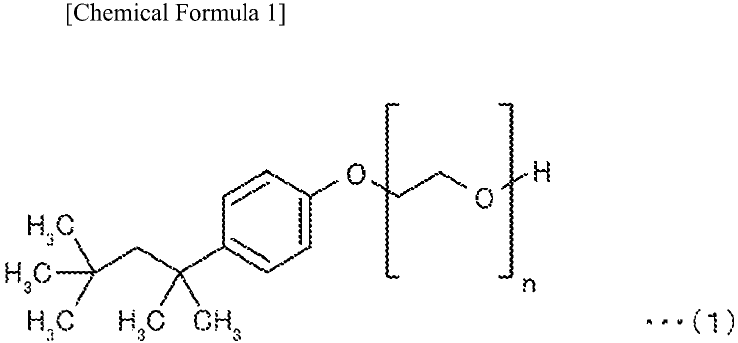

The animal may be of any species, and examples thereof comprise mammalian animals (mammals) such as pigs, avian animals (birds) such as chickens, and fish comprising Pleuronectidae fish such as yellowfin sole, Salmonidae fish such as chum salmon and Atlantic salmon, and rays (comprising, e.g., skates). Preferable examples of the animal comprise pigs, chickens, flatfishes, salmons, and rays.

The tissue of the animal is, for example, a tissue comprising proteoglycan. Examples of the tissue comprise: epithelial tissues such as skin; cartilage tissues such as cartilage; digestive organs; circulatory organs; respiratory organs; and placenta. Specific examples of the tissue of the animal comprise cartilage, fins, digestive organs, circulatory organs, respiratory organs, and ears.

Examples of the sugar chain of proteoglycan comprise chondroitin, chondroitin sulfate, dermatan sulfate (chondroitin sulfate B), heparan sulfate, heparin, and keratan sulfate. The sugar chain is preferably chondroitin sulfate.

In the production method of the present disclosure, as described above, endotoxin is reduced in proteoglycan derived from the tissue of the animal using the nonionic surfactant in the first reduction step. Specifically, in the first reduction step, for example, the phase of the nonionic surfactant is formed from the complex of the endotoxin and the nonionic surfactant to concentrate the endotoxin in the phase of the nonionic surfactant as described above. Thus, the production method of the present disclosure may comprise a formation step of bringing the nonionic surfactant and the proteoglycan derived from the tissue of the animal into contact prior to the first reduction step, thereby forming a complex of the nonionic surfactant comprising the endotoxin.

In the formation step, for example, the nonionic surfactant and the proteoglycan are preferably brought into contact in a liquid system (liquid phase) because the complex can be efficiently formed. The liquid system can be, for example, in the presence of a solvent such as an aqueous solvent. The liquid system is preferably substantially free of an organic solvent such as a basic organic solvent. The phrase "substantially free of" means that the content of the organic solvent is equal to or less than the detection limit, for example. Examples of the aqueous solvent comprise a buffer such as a phosphate buffer, a phosphate buffered saline comprising no calcium (Ca) and/or magnesium (Mg), a phosphate buffered saline, a phosphate buffered saline (PBS), a saline solution, a sodium chloride solution, and a mixture of these solutions. The aqueous solvent may comprise, for example, alcohol such as ethanol, for more efficient removal of endotoxin. When the aqueous solvent comprises alcohol, the alcohol is preferably comprised to the extent that the formation of the complex of the nonionic surfactant and the endotoxin is not inhibited. When the aqueous solvent comprises alcohol, the concentration of the alcohol is, for example, preferably more than 0 (v/v)% and less than 2 (v/v)%, more preferably about 1 (v/v)%.

In the formation step, the nonionic surfactant and the proteoglycan can be appropriately brought into contact depending on, for example, the states of the nonionic surfactant and the proteoglycan. The nonionic surfactant and the proteoglycan to be brought into contact may be, for example, solid or liquid. When the nonionic surfactant and the proteoglycan are solid, they are preferably dispersed in a solvent in advance to be brought into contact. When one of the proteoglycan or the nonionic surfactant is solid and the other is liquid, they can be brought into contact by, for example, a known solid-liquid contact method. Specifically, the contact is made by mixing or blending (hereinafter, collectively referred to as "mixing") the nonionic surfactant and the proteoglycan derived from the tissue of the animal. When the nonionic surfactant and the proteoglycan are solid, they can be brought into contact by, for example, adding the nonionic surfactant and the proteoglycan to a solvent. Thus, in the formation step, a coexisting system (a mixed system or solution) of the nonionic surfactant and the proteoglycan comprising the complex of the endotoxin and the nonionic surfactant can be prepared. In the coexisting system, the complex of the nonionic surfactant and the endotoxin preferably forms, for example, micelles. In this case, the coexisting system may comprise micelles of the nonionic surfactant, and the micelles may comprise endotoxin.

In the formation step, the coexisting system is preferably further mixed after the contact, for example. Thus, in the formation step, for example, the endotoxin and the nonionic surfactant can be promoted to form the complex in the coexisting system. The mixing may be performed by, for example, using a stirrer such as a vortex mixer, by inversion mixing, or by ultrasonic treatment, preferably by ultrasonic treatment.

In the formation step, the proteoglycan derived from the tissue of the animal may be proteoglycan obtained from the tissue of the animal by a pretreatment such as crude purification or purification, or may be a tissue of an animal comprising the proteoglycan. Examples of the pretreatment comprise, for example, pulverization, defatting, and purification of the tissue of the animal. The pulverization can be performed by, for example, cutting, crushing, or an ultrasonic treatment. The purification (extraction) can be performed by, for example, bringing the tissue of the animal or the pretreated animal tissue into contact with an extraction liquid. Examples of the extraction liquid comprise an aqueous guanidine hydrochloride solution, an aqueous acetic acid solution, an aqueous urea solution, and an aqueous magnesium chloride solution.

The coexisting system may have any pH level, preferably pH 5 to pH 7.4, preferably pH 5.5 or more (e.g., pH 5.5 to pH 7.4), or pH 7 or less (e.g., pH 5 to pH 7 or pH 5.5 to pH 7), more preferably pH 6 or more (e.g., pH 6 to pH 7.4 or pH 6 to pH 7), or pH 6.8 or less (e.g., pH 5 to pH 6.8, pH 5.5 to pH 6.8, pH 6 to pH 6.8), for example.

In the formation step, the temperature of the coexisting system (temperature) is, for example, a temperature below the cloud point of the nonionic surfactant. The temperature can be set, for example, depending on the type of the nonionic surfactant and the concentration of the nonionic surfactant in the coexisting system. When the nonionic surfactant is a POEAE-based nonionic surfactant, the temperature is, for example, higher than -10°C and 40°C or lower (more preferably higher than -10°C and lower than 22°C).

In the formation step, the contents of the nonionic surfactant and the proteoglycan in the coexisting system can be set for the content of endotoxin assumed to be comprised in the proteoglycan. Specifically, when the content of endotoxin in the proteoglycan is assumed to be relatively high, the content of the nonionic surfactant in the coexisting system can be set relatively high, for example. On the other hand, when the content of endotoxin in the proteoglycan is assumed to be relatively low, the content of the nonionic surfactant in the coexisting system can be set relatively low, for example. The contents of the nonionic surfactant and the proteoglycan in the coexisting system can be set for the content of the proteoglycan, for example. In this case, the coexisting system can comprise, for example, 700 µg to 1,400 µg (e.g., 711 µg or 736 µg) of the nonionic surfactant per gram of the proteoglycan.

The proteoglycan may be pretreated to reduce endotoxin by, for example, precipitating calcium salts, prior to the formation step. Specifically, the pretreatment can be performed by, for example, bringing the proteoglycan and a phosphate buffer into contact or mixing the proteoglycan and a phosphate buffer, and separating or removing a precipitate comprising calcium phosphate salt formed after the contact or the mixing and the endotoxin. The calcium phosphate salt adsorbs the endotoxin, for example. Thus, the production method of the present disclosure can more efficiently remove the endotoxin by comprising the pretreatment, for example. A supernatant after the removal of the precipitate comprises, for example, proteoglycan in which the endotoxin is reduced. In the pretreatment, for example, the supernatant may be recovered, and the recovered supernatant may be used as the proteoglycan in the formation step. The pretreatment preferably is performed for, for example, 2 hours or more, more preferably 4 hours to 36 hours, still more preferably 8 hours to 24 hours. The temperature for the pretreatment is preferably 18°C to 25°C, for example.

In the first reduction step, the coexisting system comprising the nonionic surfactant and the proteoglycan derived from the tissue of the animal is heated from a temperature below the cloud point of the nonionic surfactant to a temperature equal to or higher than the cloud point. Thus, in the first reduction step, a surfactant phase comprising the nonionic surfactant is formed with the endotoxin concentrated in the surfactant phase (concentration step). As described above, the endotoxin and the nonionic surfactant form a complex. Thus, in the concentration step, the endotoxin in the complex is also concentrated in the surfactant phase when the surfactant phase is formed, and the proteoglycan is comprised in a phase other than the surfactant phase. In the first reduction step, for example, the endotoxin in the proteoglycan derived from the tissue of the animal can be separated or extracted into the surfactant phase, and the proteoglycan can be separated or extracted into the phase other than the surfactant phase. When an aqueous solvent is used as the coexisting system, the phase other than the surfactant phase can be, for example, an aqueous phase.

The cloud point varies depending on the type of the nonionic surfactant. Thus, for the concentration step, the temperature below the cloud point and the temperature equal to or higher than the cloud point can be appropriately controlled depending on the type of the nonionic surfactant. The cloud point may be determined by, for example, preparing an aqueous solution comprising the nonionic surfactant at a concentration of the nonionic surfactant in the coexisting system to be used, and measuring the aqueous solution by the above-described method for measuring the cloud point, or may be determined based on a known cloud point of the nonionic surfactant. When the nonionic surfactant is a commercially available product, the cloud point of the nonionic surfactant can be determined with reference to, for example, the cloud point described in the attached manual. As a specific example, when the nonionic surfactant is polyethylene glycol tert-octylphenyl ether (degree of polymerization: 7 to 8), the cloud point is, for example, about 22°C (preferably 22°C to 25°C). Thus, when polyethylene glycol tert-octylphenyl ether (degree of polymerization: 7 to 8) is used as the nonionic surfactant, the temperature below the cloud point can be set to, for example, higher than -10°C and lower than 22°C, preferably about 0°C. The temperature equal to or higher than the cloud point is, for example, 22°C to 50°C, preferably about 40°C. When the nonionic surfactant is polyethylene glycol tert-octylphenyl ether (degree of polymerization: 1 to 5), the cloud point is, for example, about 38°C. When the nonionic surfactant is polyethylene glycol tert-octylphenyl ether (degree of polymerization: 9 to 10), the cloud point is, for example, about 64°C to 66°C.

In the concentration step, the temperature can be raised by heating the coexisting system. The coexisting system can be heated using a temperature regulator such as a heat block or a thermostat.

The rate (heating rate) of raising the temperature in the coexisting system from the temperature below the cloud point to the temperature equal to or higher than the cloud point is not particularly limited. The temperature rise (heating) may occur continuously or in stages, for example.

In the concentration step, for example, the coexisting system is preferably mixed at a temperature below the cloud point before the heating. The mixing may be performed, for example, using a stirrer such as a vortex mixer or by inversion mixing, preferably by inversion mixing. The mixing time at a temperature below the cloud point can be set, for example, within a range in which the temperature of the coexisting system or the solvent of the coexisting system is approximately uniform. The mixing time at a temperature below the cloud point is, for example, one to ten minutes.

In the concentration step, for example, the mixing is preferably performed at a temperature equal to or higher than the cloud point after the heating. The mixing may be performed, for example, using a vortex mixer or by inversion mixing, preferably by inversion mixing. The mixing time at a temperature equal to or higher than the cloud point can be set, for example, within a range in which the temperature of the solvent is approximately uniform. The mixing time at a temperature equal to or higher than the cloud point is, for example, one to ten minutes.

The first reduction step may comprise a separation step of separating the surfactant phase and a phase other than the surfactant phase. Thus, the first reduction step produces, for example, a layer structure comprising a layer of the surfactant phase and a layer of the phase other than the surfactant phase. The first reduction step comprises the separation step, allowing easy recovery or removal of at least one of the surfactant phase or the phase other than the surfactant phase in the coexisting system. Thus, the production method of the present disclosure can efficiently reduce endotoxin in the proteoglycan, for example. The separation step is preferably performed after the concentration step. The separation can be performed by a separation method capable of separating two phases of liquid, for example, by centrifugation. The phase other than the surfactant phase is, for example, an aqueous phase. When the separation step is performed by the centrifugation, the centrifugation is performed, for example, at about 9,500 × g.

Time for the separation step (separation time) can be appropriately set, for example, within a range in which the surfactant phase and the phase other than the surfactant phase can be separated. The separation time is, for example, 1 second to 60 seconds.

The temperature for the separation step (separation temperature) is, for example, a temperature at which the surfactant phase can be maintained, for example, specifically a temperature equal to or higher than the cloud point. When polyethylene glycol tert-octylphenyl ether (degree of polymerization: 7 to 8) is used as the nonionic surfactant, the separation temperature is, for example, 22°C to 40°C.

In the production method of the present disclosure, the first reduction step may comprise a first recovery step of recovering the phase other than the surfactant phase after the separation, thereby recovering proteoglycan in which the endotoxin is reduced. The recovery is performed by any method as long as the aqueous phase which is the phase other than the surfactant phase of the supernatant can be recovered. The recovery may be performed using, for example, a dropper.

In the production method of the present disclosure, for example, the first reduction step may be repeated once or more using the recovered proteoglycan as the proteoglycan derived from the tissue of the animal. In the production method of the present disclosure, for example, the amount of endotoxin in the proteoglycan can further be reduced by repeating the first reduction step once or more.

When the first reduction step is repeated once or more, the surfactants used in each first reduction step may be the same or different, for example.

In the production method of the present disclosure, when the first reduction step is repeated once or more, for example, the formation step may be performed before repeating the first reduction step. In this case, for example, the coexisting system preferably comprises the aqueous solvent in the first formation step, and comprises the aqueous solvent comprising alcohol in the second and subsequent formation steps. Thus, the production method of the present disclosure can remove endotoxin more efficiently, for example.

The production method of the present disclosure may comprise second reduction step performed on the proteoglycan obtained by the first reduction step (hereinafter, also referred to as "first proteoglycan"). Specifically, in the second reduction step, endotoxin in the first proteoglycan is reduced by treating the first proteoglycan with a chromatography carrier. In the production method of the present disclosure, for example, the second reduction step can remove endotoxin that is less likely to associate with the nonionic surfactant, reducing more endotoxin.

The second reduction step of the present disclosure is presumed to be able to reduce more endotoxin by the mechanism described below using the chromatography carrier. However, the present disclosure is not limited to the following mechanism. Gram-negative bacteria comprise endotoxins having glycolipids of various types or structures. The glycolipids of different structures have different degrees of hydrophobicity and different degrees of charge, and the endotoxins have different degrees of hydrophobicity and different degrees of charge depending on the Gram-negative bacteria from which the endotoxins are derived. Even from the Gram-negative bacteria of the same type, the glycolipids produced are not the same in structure, but vary, and the endotoxins also vary in the degree of hydrophobicity and the degree of charge. The endotoxin removable by using the nonionic surfactant and the endotoxin removable by using the chromatography carrier are presumed to have different degrees of hydrophobicity and different degrees of charge. Thus, in the present disclosure, endotoxin is reduced by the treatment with the nonionic surfactant, and then the treatment with the chromatography carrier is performed by making use of the difference in the degree of hydrophobicity and/or the degree of charge between the endotoxins removable. These treatments could reduce more endotoxin in the proteoglycan.

In the second reduction step, the first proteoglycan and the chromatography carrier can be appropriately brought into contact depending on, for example, the state of the first proteoglycan. The first proteoglycan may be, for example, proteoglycan that has gone through the first recovery step, or proteoglycan obtained after the solidification step described below performed on the proteoglycan that has gone through the first recovery step. The proteoglycan obtained after the solidification step is preferably dispersed in a solvent before the contact with the chromatography carrier. The solvent can be selected, for example, for the type of the chromatography carrier described below. As a specific example, when an anion exchange resin is used as the chromatography carrier, the solvent may be, for example, a solvent obtained by adding salt to an amine-based solvent, a Tris hydrochloric acid buffer, HEPES, an aqueous potassium chloride solution, an aqueous sodium chloride solution, or an aqueous magnesium chloride solution. When a hydrophobic interaction resin is used as the chromatography carrier, the solvent may be, for example, a solvent obtained by adding salt to an amine-based solvent, a Tris hydrochloric acid buffer, HEPES, an aqueous potassium chloride solution, an aqueous sodium chloride solution, or an aqueous magnesium chloride solution.

Examples of the chromatography resin comprise ion exchange carriers such as ion exchange resins and hydrophobic interaction carriers such as hydrophobic interaction resins. Examples of the ion exchange carriers comprise anion exchange resins and anion exchange monoliths. When the hydrophobic interaction carrier or the anion exchange carrier is used as the chromatography carrier, for example, endotoxin in the proteoglycan obtained by the first reduction step can be adsorbed to the chromatography carrier in the second reduction step. When the anion exchange carrier is used as the chromatography carrier, a fraction of proteoglycan in the proteoglycan obtained by the first reduction step can be adsorbed to the carrier. Examples of the anion exchange carrier comprise a solid phase having, as functional groups, primary, secondary, or tertiary amino groups comprising polyamine functional groups such as diethylaminoethyl (DEAE) groups and polylysine (e.g., ε-polylysine), or quaternary ammonium groups such as trimethylammonium groups and dimethylethanolammonium groups. Examples of the hydrophobic interaction carrier comprise a solid phase having, as functional groups, polyamine functional groups such as polylysine (e.g., ε-polylysine) or alkyl groups such as butyl groups, octyl groups, and octadecyl groups. The chromatography carrier may have one kind of functional group or two or more kinds of functional groups. One kind of chromatography carrier may be used alone, or two or more kinds thereof may be used in combination. The shape of the chromatography carrier can be designed for the type of the carrier, for example. When the chromatography carrier is a chromatography resin, the chromatography resin is, for example, spherical or bead-shaped. When the chromatography carrier is a monolith, the monolith is, for example, cylindrical. The chromatography carrier may be, for example, a commercially available one. Examples of the chromatography resins having the diethylaminoethyl (DEAE) groups comprise TOYOPEARL (trademark) DEAE 650M (Tosoh Corporation). Examples of the chromatography resins having polylysine comprise Cellufine ET Clean S (JNC Corporation) and Cellufine ET Clean L (JNC Corporation). Examples of the chromatography resins having the octyl groups comprise Nucleosil 100-C8 (GL-Science). Examples of the chromatography resins having the octadecyl groups comprise Nucleosil 100-C18 (GL-Science). Examples of the monolith having the quaternary ammonium groups comprise CIMmultus (trademark) QA (Sartorius AG). Examples of the monolith having the diethylaminoethyl (DEAE) groups comprise CIMmultus (trademark) DEAE (Sartorius AG). Examples of the monolith having the butyl groups comprise CIMmultus (trademark) C4-HLD and CIMmultus (trademark) C4-A (Sartorius AG).

The second reduction step can be performed for the type of the chromatography carrier, for example. When the chromatography carrier adsorbs endotoxin but does not adsorb proteoglycan, the second reduction step comprises, for example, an adsorption step of bringing the first proteoglycan and the chromatography carrier into contact, thereby adsorbing endotoxin to the chromatography carrier, and a recovery step of recovering proteoglycan in which the endotoxin is reduced (second recovery step). That is, the second reduction step can be performed in, for example, the F/T mode. When the chromatography carrier adsorbs endotoxin and proteoglycan, the second reduction step comprises, for example, an adsorption step of bringing the first proteoglycan and the chromatography carrier into contact, thereby adsorbing proteoglycan to the chromatography carrier, and a recovery step of recovering the proteoglycan adsorbed (second recovery step). That is, the second reduction step can be performed in, for example, the B/E mode. When the second reduction step is performed in the B/E mode, the recovery can also be referred to as elution.

When the second reduction step is performed in the F/T mode, a hydrophobic interaction carrier such as the hydrophobic interaction resin, for example, can be used as the chromatography carrier. In the adsorption step, the first proteoglycan and the chromatography carrier can be brought into contact by, for example, bringing a dispersion comprising the first proteoglycan (may be referred to as a "first dispersion") into contact with the chromatography carrier. Specifically, the contact can be achieved by, for example, mixing the first dispersion and the chromatography carrier. In the adsorption step, for example, the contact between the first proteoglycan and the chromatography carrier causes an interaction between endotoxin comprised in the first proteoglycan and the chromatography carrier depending on the properties of the chromatography carrier. As a result, in the adsorption step, the chromatography carrier adsorbs endotoxin. In the adsorption step, for example, substantially no interaction occurs between a fraction of proteoglycan comprised in the first proteoglycan and the chromatography carrier, or the interaction occurs sufficiently less as compared with the interaction between the endotoxin and the chromatography carrier. Thus, in the adsorption step, for example, the proteoglycan is not substantially adsorbed to the chromatography carrier, or the amount of the proteoglycan adsorbed is sufficiently lower than the amount of the endotoxin adsorbed, maintaining the proteoglycan in the dispersion.

For the solvent of the first dispersion, for example, the description of the solvent used in the second reduction step can be referred to.

In the adsorption step, the amount of proteoglycan to be brought into contact with the chromatography carrier can be set, for example, for the amount of endotoxin adsorbed to the chromatography carrier and the amount of endotoxin in the first proteoglycan. As a specific example, when Cellufine ET Clean S (JNC Corporation) or Cellufine ET Clean L (JNC Corporation), which are hydrophobic interaction resins, is used as slurry of the chromatography carrier in the adsorption step, for example, 350 µg to 550 µg of the proteoglycan is brought into contact with 1 ml of the slurry of the chromatography carrier.

When the chromatography carrier is a hydrophobic interaction resin, the conditions for the first dispersion to be brought into contact with the chromatography carrier can be set for the type of the hydrophobic interaction resin. Specifically, the conditions for the first dispersion are as follows.
Salt concentration in the first dispersion:
(in the case of NaCl) 0.1 mol/l to 0.2 mol/l or 0.15 mol/l to 0.2 mol/l

Next, in the second recovery step, for example, a liquid fraction is recovered from a mixture of the first proteoglycan dispersion and the chromatography carrier. This allows recovery of a dispersion comprising proteoglycan in which the endotoxin is reduced (second dispersion). The second dispersion can be recovered by, for example, a known solid-liquid separation method.

In the second recovery step, the chromatography carrier after the recovery of the second dispersion may be brought into contact with a washing solution to wash the chromatography carrier. In this case, in the second recovery step, the dispersion comprising proteoglycan in which endotoxin is reduced may be recovered after the washing by recovering a liquid fraction from a mixture of the washing solution and the chromatography carrier. The recovered dispersion may be recovered as the second dispersion. The proteoglycan still remains around the chromatography carrier after the second recovery step. Thus, in the second recovery step, the chromatography carrier is washed with the washing solution, allowing the proteoglycan to be extracted and recovered. In the second recovery step, for example, the chromatography carrier is washed with the washing solution, and the washing solution is recovered. This can improve the recovery rate of the proteoglycan.

A solvent of the washing solution can be, for example, the same solvent used for the first dispersion. For the conditions for the washing solution, the conditions for the first dispersion can be referred to, for example.

When the second reduction step is performed in the B/E mode, an anion exchange carrier such as an anion exchange resin can be used as the chromatography carrier. In the adsorption step, the first proteoglycan and the chromatography carrier can be brought into contact in the same manner as in the F/T mode, for example. In the adsorption step, for example, the first proteoglycan negatively charged and the endotoxin are brought into contact with the chromatography carrier to bind the first proteoglycan and the endotoxin to the chromatography carrier, depending on the properties of the chromatography carrier. As a result, the adsorption step allows the proteoglycan and the endotoxin to be adsorbed to the chromatography carrier.

In the adsorption step, the amount of proteoglycan to be brought into contact with the chromatography carrier can be set, for example, for the amount of endotoxin adsorbed to the chromatography carrier and the amount of endotoxin in the first proteoglycan. Specifically, when TOYOPEARL (trademark) DEAE-650M (Tosoh Corporation), which is an anion exchange resin, is used as slurry of the chromatography carrier in the adsorption step, for example, 350 µg to 550 µg of the proteoglycan is brought into contact with 1 ml of the slurry of the chromatography carrier.

When the chromatography carrier is an anion exchange resin, the conditions for the first dispersion to be brought into contact with the chromatography carrier can be set for the type of the anion exchange resin. Specifically, the conditions for the first dispersion are as follows.
Salt concentration in the first dispersion:
(in the case of NaCl) 0.1 mol/l to 0.2 mol/l or 0.15 mol/l to 0.2 mol/l

The proteoglycan has, for example, a sulfo group. The endotoxin has, for example, a phosphate group. Thus, the proteoglycan and the endotoxin are different in the degree of anionic charge and the degree of hydrophobicity. In the second reduction step, for example, a solvent having a higher salt concentration than the solvent used in the adsorption step is passed through the chromatography carrier after the adsorption step to preferentially elute the proteoglycan from the chromatography carrier. Thus, the second reduction step preferably comprises, for example, an elution step of eluting the proteoglycan adsorbed to the chromatography carrier after the adsorption step.

In the elution step, examples of an elution solvent comprise an aqueous potassium chloride solution, an aqueous sodium chloride solution, and an aqueous magnesium chloride solution. The elution solvent is preferably a solvent having a higher salt concentration than the solvent used in the adsorption step, for example. Specifically, the conditions for the elution solvent are as follows.
Salt concentration of elution solvent:
(in the case of NaCl) 0.4 mol/l to 2 mol/l, 0.45 mol/l to 1 mol/l, or 0.5 mol/l to 1mol/l

In the second recovery step, for example, an eluate obtained after the elution step is recovered to recover a dispersion comprising proteoglycan in which the endotoxin is reduced (second dispersion).

In the second reduction step, the chromatography carrier may be equilibrated before the adsorption step. When an ion exchange resin or a hydrophobic interaction resin is used as the chromatography carrier, the same solvent as the solvent used in the adsorption step can be used as an equilibration solution for the chromatography resin, for example.

The chromatography carrier may be packed in a column for use, for example. In this case, the adsorption step can be performed by loading the equilibration solution, the first dispersion, the washing solution, or the eluate in a column filled with the chromatography carrier.

In the production method of the present disclosure, for example, the second reduction step may be repeated once or more on the proteoglycan recovered in the second recovery step. That is, in the production method of the present disclosure, the second reduction step may be performed more than once. In the production method of the present disclosure, for example, the amount of endotoxin in the proteoglycan can be further reduced by repeating the second reduction step more than once. In this case, the description of the second reduction step can be applied by replacing "proteoglycan obtained by the first recovery step" with "proteoglycan obtained by the second recovery step."

When the second reduction step is performed more than once, the second reduction step may comprise, for example, the following (A) and (B) steps performed on the proteoglycan obtained by the first reduction step.
Step (A): an adsorption step of bringing the proteoglycan and the chromatography carrier into contact, thereby adsorbing endotoxin to the chromatography carrier and a recovery step of recovering proteoglycan in which endotoxin is reduced; and
Step (B): an adsorption step of bringing the proteoglycan and the chromatography carrier into contact, thereby adsorbing proteoglycan to the chromatography carrier and a recovery step of recovering the proteoglycan adsorbed.

The chromatography carrier used in the step (A) is preferably an ion exchange resin or a hydrophobic interaction resin. The chromatography carrier used in the step (B) is preferably an anion exchange carrier having a DEAE group, for example.

When the second reduction step is performed more than once, the second reduction step may comprise a first treatment step of treating the proteoglycan obtained by the first reduction step with the chromatography carrier and a second treatment step of treating the treated proteoglycan with the chromatography carrier. The chromatography carrier used for the first treatment step and the chromatography carrier used for the second treatment step are preferably different chromatography carriers, for example. The first treatment step and the second treatment step can be performed in the same manner as the second reduction step.

In the production method of the present disclosure, the concentration of endotoxin in proteoglycan comprised in the recovered aqueous phase may be measured after the first reduction step or the second reduction step. The endotoxin concentration can be measured by a gelation method (LAL test) using a lysate reagent prepared from a Limulus blood cell extract, or an optical quantification method (colorimetric method).

The endotoxin concentration after the first reduction step is, for example, 10 EU/mg or less, or 7 EU/mg or less. The endotoxin concentration after the second reduction step is, for example, 4 EU/mg or less or 2 EU/mg or less, preferably 1 EU/mg or less, more preferably 0.61 EU/mg or less or 0.4 EU/mg or less, still more preferably 0.04 EU/mg or less. The lower limit of the endotoxin concentration is preferably, for example, 0 EU/mg, but in practice, the endotoxin concentration is preferably higher than 0 EU/mg (about the detection limit).

In the production method of the present disclosure, the molecular weight of the proteoglycan comprised in the recovered aqueous phase may be measured after the first reduction step or the second reduction step. The molecular weight of the proteoglycan means a peak top molecular weight measured by gel permeation chromatography (GPC). The molecular weight of the proteoglycan can be measured by GPC or any other method. GPC is performed, for example, under the following conditions, and the molecular weight can be calculated by injecting the following standard samples (molecular weight markers, pullulan) individually into an HPLC system to obtain a molecular weight calibration curve.

### (Conditions for Measurement of Peak Top Molecular Weight (Mp))

HPLC system: LC-20AD (manufactured by Shimadzu Corporation)
Column: TSKgel G5000-PWXL, ø7.8 mm × 300 mm (manufactured by Tosoh Corporation)
Eluent: pH 6.8 phosphate buffer
Flow rate: 0.5 ml/min
Column temperature: 40°C
Detector: differential refractometer (RID-20A manufactured by Shimadzu Corporation)
Injection volume: 50 µL
Molecular weight marker: Shodex STANDARD P-82 (pullulan)

The proteoglycan after the first or second reduction step has a peak top molecular weight of, for example, 300,000 to 1,300,000 or 400,000 to 1,200,000.

The production method of the present disclosure may comprise a fractionation step of fractionating proteoglycan satisfying a predetermined peak top molecular weight from the recovered phase other than the surfactant phase after the first reduction step or the second reduction step. The fractionation step can be performed by any method as long as the fractionation is performed with the predetermined molecular weight of the proteoglycan maintained. The fractionation may be performed by, for example, a known method capable of fractionating a component based on the molecular weight. Specifically, the fractionation may be performed using an ultrafiltration membrane (molecular weight cut-off membrane) or a dialysis membrane, or by silica gel column chromatography, gel filtration chromatography, or ion exchange column chromatography. The molecular weight cut-off membrane can have a molecular weight cut-off value that is set for the target molecular weight, for example.

The fractionation can be performed for any amount of time (fractionation time) as long as the time is within a range that allows the fractionation with the predetermined molecular weight of the proteoglycan maintained, for example. The fractionation time is, for example, one day to two weeks.

The production method of the present disclosure may comprise a solidification step of solidifying the fractionated proteoglycan after the fractionation step. The solidification step can be performed by any method as long as the proteoglycan can be solidified with the predetermined molecular weight of the proteoglycan maintained. The solidification step can be performed by, for example, a known method capable of solidifying proteoglycan from the fractionated liquid comprising the proteoglycan, and specifically, can be performed by drying such as freeze drying or atomization drying (spray drying).

### <Agent>

In another aspect, the present disclosure provides an agent for reducing endotoxin of proteoglycan derived from a tissue of an animal. The agent of the present disclosure comprises a nonionic surfactant. The agent of the present disclosure can reduce endotoxin in proteoglycan derived from the tissue of the animal, and thus can be suitably used for the production of proteoglycan in which the endotoxin is reduced.

For the agent of the present disclosure, for example, the description of the method for producing proteoglycan of the present disclosure can be referred to.

### <Composition>

In another aspect, the present disclosure provides a composition comprising proteoglycan derived from a tissue of an animal. The composition of the present disclosure comprises proteoglycan and endotoxin, and the content ratio of the endotoxin to the composition is, for example, 4 EU/mg or less or 2 EU/mg or less, preferably 1 EU/mg or less, more preferably 0.61 EU/mg or less or 0.4 EU/mg or less, still more preferably 0.04 EU/mg or less. The lower limit of the content ratio of endotoxin is preferably, for example, 0 EU/mg, but in practice, the content ratio is preferably higher than 0 EU/mg (about the detection limit).

The composition of the present disclosure comprises, for example, proteoglycan obtained by the method for producing proteoglycan of the present disclosure. For the composition of the present disclosure, for example, the description of the method for producing proteoglycan of the present disclosure can be referred to.

### Examples

Examples of the present disclosure will be described below. Note that the present disclosure is not limited to the following examples. Commercial reagents were used according to their protocols unless otherwise indicated. Note that "mol/l" may be denoted as "M."

### [Example 1]

The production method of the present disclosure was able to reduce endotoxin from animal tissue-derived proteoglycan, with a predetermined molecular weight of the proteoglycan (about 450,000) maintained.

To a 0.2 mol/l NaCl-comprising phosphate buffer (pH 6), 7.8 mg of salmon-derived proteoglycan was added. After the addition, the mixture was stirred and sonicated at about 23°C. Then, 5 µl of Triton (trademark) X-114 was added. After the addition, the mixture was mixed by inversion at 0°C for six minutes. After the inversion mixing, the mixture was further mixed by inversion at 37°C for five minutes. After the inversion mixing, centrifugation was performed at 23°C and 10,000 rpm for 30 seconds. After the centrifugation, two layers separated in the mixture were confirmed. After the confirmation, the upper layer comprising proteoglycan was recovered and dialyzed with a molecular weight cut off (MWCO) of 12,000 to 16,000. After the dialysis, the proteoglycan was freeze-dried to obtain a proteoglycan solid. Thereafter, the endotoxin concentrations of proteoglycan before the treatment and the proteoglycan solid were measured by the LAL method using a kit (ToxinSensor Chromogenic LAL Endotoxin Assay Kit manufactured by GenScript). The proteoglycan before the treatment and the proteoglycan solid were analyzed by HPLC under the following measurement conditions.

### (Measurement Conditions of HPLC)

HPLC system: LC-20AD (manufactured by Shimadzu Corporation)
Column: TSKgel G5000-PWXL ø7.8 mm × 300 mm (manufactured by Tosoh Corporation)
Eluent: pH 6.8 phosphate buffer
Flow rate: 0.5 ml/min
Column temperature: 40°C
Detector: differential refractometer (RID-10A manufactured by Shimadzu Corporation)
Injection volume: 50 µL
Molecular weight marker: Shodex STANDARD P-82 (pullulan)

The molecular weight markers had the peak top molecular weight (Mp) and the ratio of a weight average molecular weight (Mw) to a number average molecular weight (Mn) described below.
STD P-800: Mp: 739,000, Mw/Mn: 1.24
STD P-400: Mp: 348,000, Mw/Mn: 1.33
STD P-200: Mp: 216,000, Mw/Mn: 1.22
STD P-100: Mp: 107,000, Mw/Mn: 1.12
STD P-50: Mp: 49,400, Mw/Mn: 1.08
STD P-20: Mp: 22,000, Mw/Mn: 1.08
STD P-10: Mp: 9,800, Mw/Mn: 1.07
STD P-5: Mp: 6,300, Mw/Mn: 1.09

The analysis showed that the endotoxin concentration of the proteoglycan before the treatment was 400 EU/mg, and the endotoxin concentration of the proteoglycan solid was 4 EU/mg. The peak top molecular weight of the proteoglycan before the treatment was about 450,000, and the peak top molecular weight of the proteoglycan solid was about 450,000. The results indicate that the method of the present disclosure (Example 1) was able to reduce endotoxin in the animal tissue-derived proteoglycan, with a predetermined molecular weight of proteoglycan maintained.

Samples were prepared and analyzed as Comparative Examples 1A to 1C.

### [Comparative Example 1A]

A sample of Comparative Example 1A was prepared, measured for endotoxin concentration, and analyzed by HPLC in the same manner as in Example 1 except that the 0.2 mol/l NaCl comprising phosphate buffer (pH 6) was replaced with ethanol comprising 0.001 N sodium hydroxide. The analysis showed that the sample of Comparative Example 1A was able to maintain the predetermined molecular weight of proteoglycan (about 450,000), but reduction of endotoxin was not observed.

### [Comparative Example 1B]

A sample of Comparative Example 2 was prepared, measured for endotoxin concentration, and analyzed by HPLC in the same manner as in Example 1 except that the 0.2 mol/l NaCl comprising phosphate buffer (pH 6) was replaced with ethanol comprising 0.01 N sodium hydroxide. The sample of Comparative Example 1B comprised 167.8 EU/mg of endotoxin. The results indicate that endotoxin was not reduced in the sample of Comparative Example 1B as compared with Example 1.

### [Comparative Example 1C]

A sample of Comparative Example 1C was prepared, measured for endotoxin concentration, and analyzed by HPLC in the same manner as in Example 1 except that the 0.2 mol/l NaCl comprising phosphate buffer (pH 6) was replaced with ethanol comprising 0.025 N sodium hydroxide. The sample of Comparative Example 3 comprised 33.7 EU/mg of endotoxin and had the predetermined molecular weight of proteoglycan (about 450,000). The results indicate that the sample of Comparative Example 1C was able to reduce the endotoxin concentration, but failed to maintain the predetermined molecular weight of proteoglycan.

### [Example 2A]

It was confirmed that the production method of the present disclosure was able to reduce endotoxin in animal tissue-derived proteoglycan.

To a 0.05 mol/l phosphate buffer (pH 6.0), 7.8 mg of salmon-derived proteoglycan was added.

After the addition, 5 µl of Triton (trademark) X-114 was added. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Then, the proteoglycan before the treatment and the powdered proteoglycan were measured for endotoxin concentration by the LAL method. For the LAL method, a kit (ToxinSensor Chromogenic LAL Endotoxin Assay Kit, manufactured by GenScript) was used.

The measurement showed that the endotoxin concentration of proteoglycan before the treatment was 250.7 EU/mg, and the endotoxin concentration of the powdered proteoglycan was 9.2 EU/mg. The results indicate that the method of the present disclosure (Example 2A) was able to reduce endotoxin in the animal tissue-derived proteoglycan.

### [Example 2B]

It was confirmed that the production method of the present disclosure was able to reduce endotoxin in animal tissue-derived proteoglycan.

To a 0.05 mol/l phosphate buffer (pH 6.0), 7.8 mg of salmon-derived proteoglycan was added, and the mixture was allowed to stand at 23°C for 24 hours.

After the standing, a precipitate was removed to obtain a supernatant. Then, 5 µl of Triton (trademark) X-114 was added. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 2B was measured in the same manner as in Example 2A.

The measurement showed that the endotoxin concentration of the powdered proteoglycan of Example 2B was 9.2 EU/mg. The results indicate that the method of the present disclosure (Example 2B) was able to reduce endotoxin in the animal tissue-derived proteoglycan.

A sample was prepared and analyzed as Comparative Example 2.

### [Comparative Example 2]

To a 0.05 mol/l phosphate buffer (pH 6.0), 7.8 mg of salmon-derived proteoglycan was added, and the mixture was allowed to stand at 23°C for 24 hours.

After the standing, a precipitate was removed to obtain a supernatant. To the supernatant, three times the amount of saturated ethanol was added for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the endotoxin concentration of the powdered proteoglycan of Comparative Example 2 was measured in the same manner as in Example 2A.

The measurement showed that the endotoxin concentration of the powdered proteoglycan of Comparative Example 2 was 170.1 EU/mg.

### [Example 3A]

It was confirmed that the production method of the present disclosure was able to reduce endotoxin in animal tissue-derived proteoglycan.

Powdered proteoglycan was obtained in the same manner as in Example 2A except that PBS (pH 7.2, Cat No.: 164-28713, manufactured by FUJIFILM Wako Pure Chemical Corporation) was used as a solvent of the proteoglycan solution. Thereafter, the endotoxin concentration of the powdered proteoglycan of Example 3A was measured in the same manner as in Example 2A.

The measurement showed that the endotoxin concentration of the proteoglycan before the treatment was 304.2 EU/mg, and the endotoxin concentration of the powdered proteoglycan was 21.4 EU/mg. The results indicate that the method of the present disclosure (Example 3A) was able to reduce endotoxin in the animal tissue-derived proteoglycan.

### [Example 3B]

It was confirmed that the production method of the present disclosure was able to reduce endotoxin in animal tissue-derived proteoglycan by repeating the reduction step.

Salmon-derived proteoglycan was added to PBS (pH 7.2, Cat No.: 164-28713, manufactured by FUJIFILM Wako Pure Chemical Corporation). After the addition, the mixture was stirred and sonicated at 23°C to obtain a 5 mg/ml proteoglycan solution. Then, 1 (v/v)% Triton (trademark) X-114 was added. After the addition, the mixture was allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising the proteoglycan was recovered not to comprise the solution around the boundary between the lower layer and the upper layer. To the recovered product, 1 (v/v)% Triton (trademark) X-114 was added. After the addition, the mixture was allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 3B was measured in the same manner as in Example 2A.

The measurement showed that the endotoxin concentration of the powdered proteoglycan of Example 3B was 7.8 EU/mg. The sample of Example 3B reduced the endotoxin concentration to about 1/3 of that of the sample of Example 3A. The results indicate that the production method of the present disclosure was able to further reduce the endotoxin concentration of the proteoglycan by repeating the formation step and the reduction step.

### [Example 4A]

It was confirmed that the production method of the present disclosure was able to reduce endotoxin in animal tissue-derived proteoglycan.

Powdered proteoglycan was obtained in the same manner as in Example 2A except that PBS (pH 7.2, Cat No.: 164-28713, manufactured by FUJIFILM Wako Pure Chemical Corporation) was used as a solvent of the proteoglycan solution. Thereafter, the endotoxin concentration of the powdered proteoglycan of Example 4A was measured in the same manner as in Example 2A.

The measurement showed that the endotoxin concentration of the proteoglycan before the treatment was 403.7 EU/mg, and the endotoxin concentration of the powdered proteoglycan was 14.0 EU/mg. The results indicates that the method of the present disclosure (Example 4A) was able to reduce endotoxin in the animal tissue-derived proteoglycan.

### [Example 4B]

It was confirmed that the production method of the present disclosure was able to reduce endotoxin in the animal tissue-derived proteoglycan by using a 1 (v/v)% ethanol comprising aqueous solvent in a coexisting system in the formation step of the production method of the present disclosure.

Salmon-derived proteoglycan was added to PBS (pH 7.2, Cat No.: 164-28713, manufactured by FUJIFILM Wako Pure Chemical Corporation). After the addition, the mixture was stirred and sonicated at about 23°C to obtain a 5 mg/ml proteoglycan solution. Then, 1 (v/v)% ethanol and 1 (v/v)% Triton (trademark) X-114 were added to the solution. After the addition, the mixture was allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 4B was measured in the same manner as in Example 2A.

The measurement showed that the endotoxin concentration of the powdered proteoglycan of Example 4B was 13.7 EU/mg. The results indicate that the method of the present disclosure (Example 3A) was able to reduce endotoxin in the animal tissue-derived proteoglycan by using the 1 (v/v)% ethanol-comprising aqueous solvent in the coexisting system in the formation step of the production method of the present disclosure.

### [Example 4C]

It was confirmed that the production method of the present disclosure was able to reduce endotoxin in animal tissue-derived proteoglycan by repeating the reduction step.

Powdered proteoglycan was obtained in the same manner as in Example 3B except that PBS (pH 7.2, Cat No.: 164-28713, manufactured by FUJIFILM Wako Pure Chemical Corporation) was used as a solvent of the proteoglycan solution. Thereafter, the endotoxin concentration of the powdered proteoglycan of Example 4C was measured by the LAL method in the same manner as in Example 2A.

The measurement showed that the endotoxin concentration of the powdered proteoglycan of Example 4C was 12.6 EU/mg. The results indicate that the production method of the present disclosure was able to further reduce the endotoxin concentration of the proteoglycan by repeating the reduction step.

### [Example 4D]

It was confirmed that the production method of the present disclosure was able to reduce endotoxin in animal tissue-derived proteoglycan by repeating the reduction step and by using a 1 (v/v)% ethanol-comprising aqueous solvent in a coexisting system in the second formation step of the production method of the present disclosure.

Salmon-derived proteoglycan was added to PBS (pH 7.2, Cat No.: 164-28713, manufactured by FUJIFILM Wako Pure Chemical Corporation). After the addition, the mixture was stirred and sonicated at about 23°C to obtain a 5 mg/ml proteoglycan solution. Then, 1 (v/v)% Triton (trademark) X-114 was added. After the addition, the mixture was allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising the proteoglycan was recovered not to comprise the solution around the boundary between the lower layer and the upper layer. Then, ethanol in an amount of 1% and 1 (v/v)% Triton (trademark) X-114 were added to the recovered solution. After the addition, the mixture was allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 4D was measured by the LAL method in the same manner as in Example 2A.

The measurement showed that the endotoxin concentration of the powdered proteoglycan of Example 4D was 9.7 EU/mg. The results indicate that the production method of the present disclosure was able to further reduce the endotoxin concentration of the proteoglycan by repeating the reduction step and using the alcohol-comprising aqueous solvent in the coexisting system in the second formation step.

### [Example 5A]

To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the proteoglycan before the treatment and the powdered proteoglycan of Example 5A were measured for endotoxin concentration in the same manner as in Example 2A.

The measurement showed that the endotoxin concentration of the proteoglycan before the treatment was 362.6 EU/mg, and the endotoxin concentration of the powdered proteoglycan of Example 5A was 10.5 EU/mg. The results indicate that the production method of the present disclosure was able to reduce the endotoxin concentration of the proteoglycan.

### [Example 5B]

To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising the proteoglycan was recovered not to comprise the solution around the boundary between the lower layer and the upper layer. To the recovered product, 1 (v/v)% Triton (trademark) X-114 was added. After the addition, the mixture was allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 5B was measured in the same manner as in Example 2A.

The measurement showed that the endotoxin concentration of the powdered proteoglycan of Example 5B was 9.7 EU/mg. The results indicate that the production method of the present disclosure was able to reduce the endotoxin concentration of the proteoglycan.

### [Example 5C]

To a 0.05 mol/l phosphate buffer (pH 6.0), salmon-derived proteoglycan was added to prepare a 5 mg/ml proteoglycan solution.

After the preparation, Triton (trademark) X-45 in an amount of 1% was added to the solution. After the addition, the mixture was stirred and allowed to stand at 4°C for 10 minutes. After the standing, the mixture was allowed to stand at 40°C for 10 minutes. After the standing, the mixture was centrifuged at 37°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 37°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 5C was measured in the same manner as in Example 2A.

The measurement showed that the endotoxin concentration of the powdered proteoglycan of Example 5C was 6.9 EU/mg. The results indicate that the production method of the present disclosure was able to reduce the endotoxin concentration of the proteoglycan.

### [Example 5D]

To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising the proteoglycan was recovered not to comprise the solution around the boundary between the lower layer and the upper layer. To the recovered solution, a 1 (v/v)% mixture of Triton (trademark) X-45 and Triton (trademark) X-100 mixed in a ratio of 3:10 was added. After the addition, the mixture was allowed to stand at 4°C for 10 minutes. After the standing, the mixture was allowed to stand at 40°C for 10 minutes. After the standing, the mixture was centrifuged at 37°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 37°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 5D was measured in the same manner as in Example 2A.

The measurement showed that the concentration of endotoxin in the powdered proteoglycan of Example 5D was 9.2 EU/mg. The results indicate that the production method of the present disclosure was able to reduce the endotoxin concentration of the proteoglycan.

### [Example 5E]

To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising the proteoglycan was recovered not to comprise the solution around the boundary between the lower layer and the upper layer. To the recovered solution, a 1 (v/v)% mixture of Triton (trademark) X-45 and Triton (trademark) X-100 mixed in a ratio of 3:20 was added. After the addition, the mixture was allowed to stand at 4°C for 10 minutes. After the standing, the mixture was allowed to stand at 40°C for 10 minutes. After the standing, the mixture was centrifuged at 37°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 37°C and 9,060 × g for three minutes. After the centrifugation, saturated ethanol was removed, and the residue was dried under vacuum to obtain powdered proteoglycan. Thereafter, the endotoxin concentration of the powdered proteoglycan of Example 5E was measured in the same manner as in Example 2A.

The measurement showed that the endotoxin concentration of the powdered proteoglycan of Example 5E was 6.9 EU/mg. The results indicate that the production method of the present disclosure was able to reduce the endotoxin concentration of the proteoglycan.

### [Example 6A]

It was confirmed that the production method of the present disclosure was able to reduce endotoxin in the animal tissue-derived proteoglycan.
To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the powdered proteoglycan was chromatographed using an anion exchange resin. Specifically, the powdered proteoglycan was dissolved in 0.2 mol/l NaCl. After the dissolution, the obtained sample was charged in a DEAE M column (manufactured by Tosoh Corporation), and NaCl dissolving the proteoglycan was allowed to pass through the column. After the passage, the column was washed with 0.2 mol/l NaCl. After the washing, 0.5 mol/l NaCl was allowed to pass through the column to elute proteoglycan. The eluate comprising the proteoglycan eluted as described above was desalted and freeze-dried in a predetermined manner to obtain powdered proteoglycan. Thereafter, the endotoxin concentration of the powdered proteoglycan of Example 6A was measured in the same manner as in Example 2A.

The measurement showed that the endotoxin concentration of the proteoglycan treated with Triton (trademark) X-114 and chromatographed with DEAE was 0.83 EU/mg. The results indicate that the production method of the present disclosure was able to reduce the endotoxin concentration of the proteoglycan. Although proteoglycan was eluted by passing 0.5 mol/l NaCl through the column in Example 6A, it was confirmed that proteoglycan could be eluted in the same manner when 1 mol/l or 2 mol/l NaCl was used.

### [Example 6B]

It was confirmed that the production method of the present disclosure was able to reduce endotoxin in the animal tissue-derived proteoglycan.

To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the powdered proteoglycan was chromatographed using an anion exchange resin. Specifically, the powdered proteoglycan was dissolved in 0.2 mol/l NaCl. After the dissolution, the sample was charged in a column of Cellufine (trademark) ET Clean S (manufactured by JNC Corporation), and NaCl dissolving the proteoglycan was allowed to pass through the column to recover a flow-through component. After the passage, the column was washed with 0.2 mol/l NaCl, and a flow-through component was recovered. Thereafter, the flow-through component comprising proteoglycan was desalted and freeze-dried in a predetermined manner to obtain powdered proteoglycan. Thereafter, the endotoxin concentration of the powdered proteoglycan of Example 6B was measured in the same manner as in Example 2A.

The measurement showed that the endotoxin concentration of the proteoglycan treated with Triton (trademark) X-114 and chromatographed with polylysine was 1.93 EU/mg. The results indicate that the production method of the present disclosure was able to reduce the endotoxin concentration of the proteoglycan.

### [Example 6C]

It was confirmed that the production method of the present disclosure was able to reduce endotoxin in the animal tissue-derived proteoglycan.

To a 0.05 mol/l phosphate buffer (pH 6.0), salmon-derived proteoglycan was added to prepare a 5 mg/ml proteoglycan solution.

After the preparation, Triton (trademark) X-114 in an amount of 1% was added to the solution. After the addition, the mixture was stirred and allowed to stand on ice for 10 minutes. After the standing, the mixture was allowed to stand at 40°C for 10 minutes. After the standing, the mixture was centrifuged at 23°C and 9,060 × g for three minutes. After the centrifugation, two layers separated in the mixture were confirmed. After the confirmation, the upper layer comprising proteoglycan was recovered, and three times the amount of saturated ethanol was added to the recovered product for crystallization. After the crystallization, saturated ethanol was removed, and saturated ethanol was added again. After the addition, the mixture was centrifuged at 23°C and 9,060 × g for three minutes. After the centrifugation, saturated ethanol was removed, and the residue was dried under vacuum to obtain powdered proteoglycan. Thereafter, the powdered proteoglycan was chromatographed using an anion exchange resin. Specifically, the powdered proteoglycan was dissolved in 0.2 mol/l NaCl. After the dissolution, the sample was charged in a column of Cellufine (trademark) ET Clean L (manufactured by JNC Corporation), and NaCl dissolving the proteoglycan was allowed to pass through the column to recover a flow-through component. After the passage, the column was washed with 0.2 mol/l NaCl, and a flow-through component was recovered. Thereafter, the flow-through component comprising proteoglycan was desalted and freeze-dried in a predetermined manner to obtain powdered proteoglycan. Thereafter, the endotoxin concentration of the powdered proteoglycan of Example 6C was measured in the same manner as in Example 2A.

The measurement showed that the endotoxin concentration of the proteoglycan treated with Triton (trademark) X-114 and chromatographed with polylysine was 1.93 EU/mg. The results indicate that the production method of the present disclosure was able to reduce the endotoxin concentration of the proteoglycan.

### [Comparative Example 6A]

Salmon-derived proteoglycan was chromatographed using an anion exchange resin. Specifically, the salmon-derived proteoglycan was dissolved in 0.2 mol/l NaCl. After the dissolution, the obtained sample was charged in a DEAEM column (manufactured by Tosoh Corporation), and NaCl dissolving the proteoglycan was allowed to pass through the column. After the passage, the column was washed with 0.2 mol/l NaCl. After the washing, 0.5 mol/l NaCl was allowed to pass through the column to elute proteoglycan. The eluate comprising the proteoglycan eluted as described above was desalted and freeze-dried in a predetermined manner to obtain powdered proteoglycan. Thereafter, the endotoxin concentration of the powdered proteoglycan of Comparative Example 6A was measured in the same manner as in Example 2A.

The measurement showed that the endotoxin concentration of the proteoglycan just chromatographed with DEAE was 90.6 EU/mg. The results indicate that the production method of the present disclosure was able to reduce the endotoxin concentration of the proteoglycan.

### [Comparative Example 6B]

Salmon-derived proteoglycan was chromatographed using an anion exchange resin. Specifically, the salmon-derived proteoglycan was dissolved in 0.2 mol/l NaCl. After the dissolution, the sample was charged in a column of Cellufine (trademark) ET Clean S (manufactured by JNC Corporation), and NaCl dissolving the proteoglycan was allowed to pass through the column to recover a flow-through component. After the passage, the column was washed with 0.2 mol/l NaCl, and a flow-through component was recovered. Thereafter, the flow-through component comprising proteoglycan was desalted and freeze-dried in a predetermined manner to obtain powdered proteoglycan. Thereafter, the endotoxin concentration of the powdered proteoglycan of Comparative Example 6B was measured in the same manner as in Example 2A.

The measurement showed that the endotoxin concentration of the proteoglycan just chromatographed with polylysine was 122.2 EU/mg.

### [Comparative Example 6C]

Salmon-derived proteoglycan was chromatographed using an anion exchange resin. Specifically, the proteoglycan was dissolved in 0.2 mol/l NaCl. After the dissolution, the sample was charged in a column of Cellufine (trademark) ET Clean L (manufactured by JNC Corporation), and NaCl dissolving the proteoglycan was allowed to pass through the column to recover a flow-through component. After the passage, the column was washed with 0.2 mol/l NaCl, and a flow-through component was recovered. Thereafter, the flow-through component comprising proteoglycan was desalted and freeze-dried in a predetermined manner to obtain powdered proteoglycan. Thereafter, the endotoxin concentration of the powdered proteoglycan of Comparative Example 6C was measured in the same manner as in Example 2A.

The measurement showed that the endotoxin concentration of the proteoglycan just chromatographed with polylysine was 101.7 EU/mg.

Although the present disclosure has been described with reference to the embodiments and the examples, the present disclosure is not limited to the embodiments and the examples. Various modifications that can be understood by those skilled in the art can be made to the configuration and details of the present disclosure within the scope of the present disclosure.

The contents described in all patents, patent applications, and documents cited in the present application are incorporated herein by reference in their entirety.

This application claims priority to Japanese Patent Application No. 2023-022139 filed on February 16, 2023, Japanese Patent Application No. 2023-127199 filed on August 3, 2023, and Japanese Patent Application No. 2023-203052 filed on November 30, 2023, the entire disclosure of which is incorporated herein by reference.

### <Supplementary Note>

Some or all of the above-described embodiments and examples can be modified as in the following supplementary notes, but are not limited to them.

### <Agent>

### (Supplementary Note A1)

An agent for reducing endotoxin in proteoglycan derived from a tissue of an animal, the agent comprising a nonionic surfactant.

### (Supplementary Note A2)

The agent of Supplementary Note A1, wherein the nonionic surfactant is a polyoxyethylene alkyl ether (POEAE)-based nonionic surfactant.

### (Supplementary Note A3)

The agent of Supplementary Note A1 or A2, wherein the nonionic surfactant is polyethylene glycol tert-octylphenyl ether.

### (Supplementary Note A4)

The agent of any one of Supplementary Notes A1 to A3, wherein the animal is selected from the group consisting of salmons, pigs, birds, flatfishes, and rays.

### <Method for Producing Proteoglycan>

### (Supplementary Note A5)

A method for producing proteoglycan, comprising: a first reduction step of reducing endotoxin in proteoglycan derived from a tissue of an animal by using a nonionic surfactant; and a second reduction step of treating the proteoglycan with a chromatography carrier, thereby reducing endotoxin in the proteoglycan.

### (Supplementary Note A6)

The method of Supplementary Note A5, wherein the second reduction step comprises: an adsorption step of bringing the proteoglycan obtained by the first reduction step and the chromatography carrier into contact, thereby adsorbing endotoxin to the chromatography carrier; and a recovery step of recovering proteoglycan in which endotoxin is reduced.

### (Supplementary Note A7)

The method of Supplementary Note A5, wherein the second reduction step comprises: an adsorption step of bringing the proteoglycan obtained by the first reduction step and the chromatography carrier into contact, thereby adsorbing the proteoglycan to the chromatography carrier; and a recovery step of recovering the proteoglycan adsorbed.

### (Supplementary Note A8)

The method of any one of Supplementary Notes A5 to A7, wherein the second reduction step is performed more than once.

### (Supplementary Note A9)

The method of any one of Supplementary Notes A5 to A8, wherein the second reduction step comprises the following steps (A) and (B) performed on the proteoglycan obtained by the first reduction step:
the step (A): an adsorption step of bringing the proteoglycan and the chromatography carrier into contact, thereby adsorbing endotoxin to the chromatography carrier and a recovery step of recovering proteoglycan in which endotoxin is reduced.
the step (B): an adsorption step of bringing the proteoglycan and the chromatography carrier into contact, thereby adsorbing the proteoglycan to the chromatography carrier; and a recovery step of recovering the proteoglycan adsorbed.

### (Supplementary Note A10)

The method of Supplementary Note A8 or A9, wherein the second reduction step comprises: a first treatment step of treating the proteoglycan obtained by the first reduction step with the chromatography carrier; and a second treatment step of treating the treated proteoglycan with the chromatography carrier, the chromatography carrier is an ion exchange carrier or a hydrophobic interaction carrier, and the chromatography carrier used for the first treatment step and the chromatography carrier used for the second treatment step are different chromatography carriers.

### (Supplementary Note A11)

The method of any one of Supplementary Notes A5 to A9, wherein the chromatography carrier is a chromatography resin.

### (Supplementary Note A12)

The method of Supplementary Note A11, wherein the chromatography resin is an ion exchange resin and/or a hydrophobic interaction resin.

### (Supplementary Note A13)

The method of Supplementary Note A12, wherein the ion exchange resin is an anion exchange resin.

### (Supplementary Note A14)

The method of Supplementary Note A13, wherein the anion exchange resin is a resin having a diethylaminoethyl group as a functional group.

### (Supplementary Note A15)

The method of Supplementary Note A12, wherein the hydrophobic interaction resin is a resin having a polyamine functional group, an octyl group, and/or an octadecyl group as a functional group.

### (Supplementary Note A16)

The method of any one of Supplementary Notes A5 to A15, wherein the first reduction step comprises: a concentration step of heating a coexisting system comprising the nonionic surfactant and the proteoglycan derived from the tissue of the animal from a temperature below a cloud point of the nonionic surfactant to a temperature equal to or higher than the cloud point, thereby forming a surfactant phase comprising the nonionic surfactant and concentrating the endotoxin in the surfactant phase; and a recovery step of recovering a phase other than the surfactant phase, thereby recovering proteoglycan in which the endotoxin is reduced.

### (Supplementary Note A17)

The method of Supplementary Note A16, wherein the first reduction step comprises a separation step of separating the surfactant phase and the phase other than the surfactant phase, and the recovery step comprises recovering the phase other than the surfactant phase after the separation.

### (Supplementary Note A18)

The method of Supplementary Note A16 or A17, wherein the phase other than the surfactant phase is an aqueous phase.

### (Supplementary Note A19)

The method of Supplementary Note A17 or A18, wherein the separation is performed by centrifugation.

### (Supplementary Note A20)

The method of any one of Supplementary Notes A16 to A19, wherein the coexisting system comprises a micelle of the nonionic surfactant, and the micelle comprises the endotoxin.

### (Supplementary Note A21)

The method of any one of Supplementary Notes A5 to A20, further comprising: a formation step of bringing the nonionic surfactant and the proteoglycan derived from the tissue of the animal into contact prior to the first reduction step, thereby forming a micelle of the nonionic surfactant comprising the endotoxin, wherein in the concentration step, the surfactant phase is formed from the micelle, thereby concentrating the endotoxin in the surfactant phase.

### (Supplementary Note A22)

The method of any one of Supplementary Notes A5 to A21, wherein the nonionic surfactant is a polyoxyethylene alkyl ether (POEAE)-based nonionic surfactant.

### (Supplementary Note A23)

The method of any one of Supplementary Notes A5 to A22, wherein the nonionic surfactant is polyethylene glycol tert-octylphenyl ether.

### (Supplementary Note A24)

The method of any one of Supplementary Notes A5 to A23, wherein the proteoglycan after the second reduction step has a peak top molecular weight of 300,000 to 1,300,000.

### (Supplementary Note A25)

The method of any one of Supplementary Notes A5 to A24, wherein a sugar chain of the proteoglycan is chondroitin sulfate.

### (Supplementary Note A26)

The method of any of Supplementary Notes A5 to A25, wherein the animal is selected from the group consisting of salmons, pigs, birds, flatfishes, and rays.

### <Method for Producing Proteoglycan>

### (Supplementary Note B1)

A method for producing proteoglycan, comprising: a reduction step of reducing endotoxin in proteoglycan derived from a tissue of an animal by using a nonionic surfactant.

### (Supplementary Note B2)

The method of Supplementary Note B1, wherein the reduction step comprises: a concentration step of heating a coexisting system comprising the nonionic surfactant and the proteoglycan derived from the tissue of the animal from a temperature below a cloud point of the nonionic surfactant to a temperature equal to or higher than the cloud point, thereby forming a surfactant phase comprising the nonionic surfactant and concentrating the endotoxin in the surfactant phase; and a recovery step of recovering a phase other than the surfactant phase, thereby recovering proteoglycan in which the endotoxin is reduced.

### (Supplementary Note B3)

The method of Supplementary Note B2, wherein the reduction step comprises a separation step of separating the surfactant phase and a phase other than the surfactant phase, and the recovery step comprises recovering the phase other than the surfactant phase after the separation.

### (Supplementary Note B4)

The method of Supplementary Note B3, wherein the phase other than the surfactant phase is an aqueous phase.

### (Supplementary Note B5)

The method of Supplementary Note B3 or B4, wherein the separation is performed by centrifugation.

### (Supplementary Note B6)

The method of any one of Supplementary Notes B2 to B5, wherein the coexisting system comprises a micelle of the nonionic surfactant, and the micelle comprises the endotoxin.

### (Supplementary Note B7)

The method of any one of Supplementary Notes B2 to B6, further comprising: a formation step of bringing the nonionic surfactant and the proteoglycan derived from the animal tissue into contact prior to the reduction step, thereby forming a micelle of the nonionic surfactant comprising the endotoxin, wherein in the concentration step, the surfactant phase is formed from the micelle, thereby concentrating the endotoxin in the surfactant phase.

### (Supplementary Note B8)

The method of any one of Supplementary Notes B1 to B7, wherein the nonionic surfactant is a polyoxyethylene alkyl ether (POEAE)-based nonionic surfactant.

### (Supplementary Note B9)

The method of any one of Supplementary Notes B1 to B8, wherein the nonionic surfactant is polyethylene glycol tert-octylphenyl ether.

### (Supplementary Note B10)

The method of any one of Supplementary Notes B1 to B9, wherein the proteoglycan after the reduction step has a peak top molecular weight of 300,000 to 1,300,000.

### (Supplementary Note B11)

The method of any one of Supplementary Notes B1 to B10, wherein a sugar chain of the proteoglycan is chondroitin sulfate.

### (Supplementary Note B12)

The method of any one of Supplementary Note B1 to B11, wherein the animal is selected from the group consisting of salmons, pigs, birds, flatfishes, and rays.

### <Composition>

### (Supplementary Note B13)

A composition comprising proteoglycan derived from a tissue of an animal, wherein the composition comprises the proteoglycan and endotoxin, and a content ratio of the endotoxin to the composition is 4 EU/mg or less.

### (Supplementary Note B14)

The composition of Supplementary Note B13, wherein the proteoglycan has a peak top molecular weight of 300,000 to 1,300,000.

### (Supplementary Note B15)

The composition of Supplementary Note B13 or B14, wherein a sugar chain of the proteoglycan is chondroitin sulfate.

### (Supplementary Note B16)

The composition of any one of Supplementary Notes B13 to B15, wherein the animal is selected from the group consisting of salmons, pigs, birds, flatfishes, and rays.

### INDUSTRIAL APPLICABILITY

As can be seen in the foregoing, the present disclosure can provide an agent capable of reducing endotoxin and used for reducing endotoxin in animal tissue-derived proteoglycan, and a method for producing proteoglycan and capable of reducing endotoxin. Thus, the present disclosure is extremely useful in, for example, pharmaceutical fields.

## Claims

1. An agent for reducing endotoxin in proteoglycan derived from a tissue of an animal, the agent comprising a nonionic surfactant.

2. The agent of claim 1, wherein
the nonionic surfactant is a polyoxyethylene alkyl ether (POEAE)-based nonionic surfactant.

3. The agent of claim 1 or 2, wherein
the nonionic surfactant is polyethylene glycol tert-octylphenyl ether.

4. The agent of any one of claims 1 to 3, wherein
the animal is selected from the group consisting of salmons, pigs, birds, flatfishes, and rays.

5. A method for producing proteoglycan, comprising:
a first reduction step of reducing endotoxin in proteoglycan derived from a tissue of an animal by using a nonionic surfactant; and
a second reduction step of treating the proteoglycan with a chromatography carrier, thereby reducing endotoxin in the proteoglycan.

6. The method of claim 5, wherein
the second reduction step comprises:
an adsorption step of bringing the proteoglycan obtained by the first reduction step and the chromatography carrier into contact, thereby adsorbing endotoxin to the chromatography carrier; and
a recovery step of recovering proteoglycan in which the endotoxin is reduced.

7. The method of claim 5, wherein
the second reduction step comprises:
an adsorption step of bringing the proteoglycan obtained by the first reduction step and the chromatography carrier into contact, thereby adsorbing the proteoglycan to the chromatography carrier; and
a recovery step of recovering the proteoglycan adsorbed.

8. The method of any one of claims 5 to 7, wherein
the second reduction step is performed more than once.

9. The method of any one of claims 5 to 8, wherein
the second reduction step comprises the following steps (A) and (B) performed on the proteoglycan obtained by the first reduction step:
the step (A): an adsorption step of bringing the proteoglycan and the chromatography carrier into contact, thereby adsorbing endotoxin to the chromatography carrier and a recovery step of recovering proteoglycan in which the endotoxin is reduced; and
the step (B): an adsorption step of bringing the proteoglycan and the chromatography carrier into contact, thereby adsorbing the proteoglycan to the chromatography carrier and a recovery step of recovering the proteoglycan adsorbed.

10. The method of claim 8 or 9, wherein
the second reduction step comprises:
a first treatment step of treating the proteoglycan obtained by the first reduction step with the chromatography carrier; and
a second treatment step of treating the treated proteoglycan with the chromatography carrier,
the chromatography carrier is an ion exchange carrier or a hydrophobic interaction carrier, and
the chromatography carrier used for the first treatment step and the chromatography carrier used for the second treatment step are different chromatography carriers.

11. The method of any one of claims 5 to 9, wherein
the chromatography carrier is a chromatography resin.

12. The method of claim 11, wherein
the chromatography resin is an ion exchange resin and/or a hydrophobic interaction resin.

13. The method of claim 12, wherein
the ion exchange resin is an anion exchange resin.

14. The method of claim 13, wherein
the anion exchange resin is a resin having a diethylaminoethyl group as a functional group.

15. The method of claim 12, wherein
the hydrophobic interaction resin is a resin having a polyamine functional group, an octyl group, and/or an octadecyl group as a functional group.

16. The method of any one of claims 5 to 15, wherein
the first reduction step comprises:
a concentration step of heating a coexisting system comprising the nonionic surfactant and the proteoglycan derived from the tissue of the animal from a temperature below a cloud point of the nonionic surfactant to a temperature equal to or higher than the cloud point, thereby forming a surfactant phase comprising the nonionic surfactant and concentrating the endotoxin in the surfactant phase; and
a recovery step of recovering a phase other than the surfactant phase, thereby recovering proteoglycan in which the endotoxin is reduced.

17. The method of claim 16, wherein
the first reduction step comprises a separation step of separating the surfactant phase and the phase other than the surfactant phase, and
the recovery step comprises recovering the phase other than the surfactant phase after the separation.

18. The method of claim 16 or 17, wherein
the phase other than the surfactant phase is an aqueous phase.

19. The method of claim 17 or 18, wherein
the separation is performed by centrifugation.

20. The method of any one of claims 16 to 19, wherein
the coexisting system comprises a micelle of the nonionic surfactant, and
the micelle comprises the endotoxin.

21. The method of any one of claims 5 to 20, further comprising:
a formation step of bringing the nonionic surfactant and the proteoglycan derived from the tissue of the animal into contact prior to the first reduction step, thereby forming a micelle of the nonionic surfactant comprising the endotoxin, wherein
in the concentration step, the surfactant phase is formed from the micelle, thereby concentrating the endotoxin in the surfactant phase.

22. The method of any one of claims 5 to 21, wherein
the nonionic surfactant is a polyoxyethylene alkyl ether (POEAE)-based nonionic surfactant.

23. The method of any one of claims 5 to 22, wherein
the nonionic surfactant is polyethylene glycol tert-octylphenyl ether.

24. The method of any one of claims 5 to 23, wherein
the proteoglycan after the second reduction step has a peak top molecular weight of 300,000 to 1,300,000.

25. The method of any one of claims 5 to 24, wherein
a sugar chain of the proteoglycan is chondroitin sulfate.

26. The method of any one of claims 5 to 25, wherein
the animal is selected from the group consisting of salmons, pigs, birds, flatfishes, and rays.

27. A method for producing proteoglycan, comprising:
a reduction step of reducing endotoxin in proteoglycan derived from a tissue of an animal by using a nonionic surfactant.

28. The method of claim 27, wherein
the reduction step comprises:
a concentration step of heating a coexisting system comprising the nonionic surfactant and the proteoglycan derived from the tissue of the animal from a temperature below a cloud point of the nonionic surfactant to a temperature equal to or higher than the cloud point, thereby forming a surfactant phase comprising the nonionic surfactant and concentrating the endotoxin in the surfactant phase; and
a recovery step of recovering a phase other than the surfactant phase, thereby recovering proteoglycan in which the endotoxin is reduced.

29. The method of claim 28, wherein
the reduction step comprises a separation step of separating the surfactant phase and a phase other than the surfactant phase, and
the recovery step comprises recovering the phase other than the surfactant phase after the separation.

30. The method of claim 29, wherein
the phase other than the surfactant phase is an aqueous phase.

31. The method of claim 29 or 30, wherein
the separation is performed by centrifugation.

32. The method of any one of claims 28 to 31, wherein
the coexisting system comprises a micelle of the nonionic surfactant, and
the micelle comprises the endotoxin.

33. The method of any one of claims 28 to 32, further comprising:
a formation step of bringing the nonionic surfactant and the proteoglycan derived from the tissue of the animal into contact prior to the reduction step, thereby forming a micelle of the nonionic surfactant comprising the endotoxin, wherein
in the concentration step, the surfactant phase is formed from the micelle, thereby concentrating the endotoxin in the surfactant phase.

34. The method of any one of claims 27 to 33, wherein
the nonionic surfactant is a polyoxyethylene alkyl ether (POEAE)-based nonionic surfactant.

35. The method of any one of claims 27 to 34, wherein
the nonionic surfactant is polyethylene glycol tert-octylphenyl ether.

36. The method of any one of claims 27 to 35, wherein
the proteoglycan after the reduction step has a peak top molecular weight of 300,000 to 1,300,000.

37. The method of any one of claims 27 to 36, wherein
a sugar chain of the proteoglycan is chondroitin sulfate.

38. The method of any one of claims 27 to 37, wherein
the animal is selected from the group consisting of salmons, pigs, birds, flatfishes, and rays.

39. A composition comprising proteoglycan derived from a tissue of an animal, wherein
the composition comprises the proteoglycan and endotoxin, and
a content ratio of the endotoxin to the composition is 4 EU/mg or less.

40. The composition of claim 39, wherein
the proteoglycan has a peak top molecular weight of 300,000 to 1,300,000.

41. The composition of claim 39 or 40, wherein
a sugar chain of the proteoglycan is chondroitin sulfate.

42. The composition of any one of claims 39 to 41, wherein
the animal is selected from the group consisting of salmons, pigs, birds, flatfishes, and rays.
